(19) Europäisches Patentamt / European Patent Office / Office européen des brevets

(11) **EP 4 548 887 B1**

(12) **EUROPEAN PATENT SPECIFICATION**

(45) Date of publication and mention
of the grant of the patent:
**17.09.2025 Bulletin 2025/38**

(21) Application number: **23206793.4**

(22) Date of filing: **30.10.2023**

(51) International Patent Classification (IPC):
*A61F 13/42* (2006.01)      *A61F 13/514* (2006.01)
*G01N 27/22* (2006.01)      *G01N 27/12* (2006.01)

(52) Cooperative Patent Classification (CPC):
**A61F 13/42; A61F 13/51458; A61F 13/5148;**
**G01N 27/121; G01N 27/225;** A61F 2013/424

(54) **SMART ABSORBENT ARTICLES WITH AUTOMATED EXUDATE DETECTION**

INTELLIGENTE SAUGFÄHIGE ARTIKEL MIT AUTOMATISCHER EXSUDATERKENNUNG

ARTICLES ABSORBANTS INTELLIGENTS À DÉTECTION AUTOMATISÉE D'EXSUDAT

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(43) Date of publication of application:
**07.05.2025 Bulletin 2025/19**

(73) Proprietors:
• **Ontex BV**
**9255 Buggenhout (BE)**
• **Ontex Group NV**
**9320 Erembodegem (BE)**

(72) Inventors:
• **Hellmold, Jens**
**59269 Beckum (DE)**
• **PEPERMANS, Manu**
**2018 Antwerpen (BE)**

(74) Representative: **Macchetta, Andrea**
**Ontex BV**
**Korte Keppestraat 21**
**9320 Erembodegem-Aalst (BE)**

(56) References cited:
EP-A1- 3 824 856          WO-A1-2019/107282
WO-A1-2022/136295      US-A1- 2012 310 190
US-A1- 2022 354 708

## Description

### TECHNICAL FIELD

The present disclosure is directed to an absorbent article for personal hygiene (such as baby or adult diapers, pants or incontinence briefs or pads) and a system for monitoring thereof.

### BACKGROUND

Absorbent articles for personal hygiene are designed to absorb and contain bodily exudates, such as a large quantity of urine. Non-limiting examples of disposable absorbent articles include diapers, pants, training pants, pADL, adult incontinence products, and feminine hygiene products (including, for example, sanitary napkins and tampons). Other examples of disposable absorbent articles include bandages and wound dressings. In some embodiments, for example, an absorbent article comprises several layers providing different functions, for example a topsheet, a backsheet and in-between an absorbent core, among other layers.

The function of the absorbent core is to absorb and retain the exudates for a prolonged amount of time, for example overnight for a diaper, minimize re-wet to keep the wearer dry and avoid soiling of clothes or bed sheets. The majority of currently marketed absorbent articles comprise as absorbent material a blend of comminuted wood pulp with superabsorbent polymers (SAP) in particulate form, also called absorbent gelling materials (AGM), see for example U.S. Pat. No. 5,151,092 (Buell). Absorbent articles having a core consisting essentially of SAP as absorbent material (so called "airfelt-free" cores) have also been proposed but are less common than traditional mixed cores (see e.g. WO2008/155699 and WO2012/052172).

A number of disclosures exist (see for example EP2496197B1, EP2739254B1, and EP2582341B1) directed to sensors to sense a condition such as temperature from body or moisture from incontinence. The sensor comprises a signal processing unit, a transmitter and a power supply, typically in form of a battery. These elements are arranged on a flexible substrate in low profile enabling disposition adjacent to the human body. A complex series of mathematical and statistical manipulations are then needed in order to determine wetness events and wetness levels.

While such devices allow monitoring conditions of the human body and can also be used as a moisture sensor, it represents also relatively costly solution. It would not be seen appropriate to dispose of the sensor together with a (disposable) absorbent article. If the sensor, however, is to be reused, the sensing area has potentially been exposed to moisture. Therefore this concept does not allow for simple usage.

Examples of articles that provide improvements to the above drawbacks are disclosed in EP3415130, WO/2018/228822, WO/2018/229017, EP3461257, and EP3451988.

WO2022/136295 A1 relates to a system for sensing diaper content comprising a diaper, an outer coupling layer and a sensing circuitry. The diaper comprises a circuitry layer having inner input capacitor plates, tracks arranged to provide a resistive coupling between the inner capacitor plates dependent on the content, and inner output capacitor plates in galvanic contact with the respective inner input capacitor plates. The sensing circuitry is arranged to apply an input signal onto the outer input capacitor plates, and to measure an output signal from the outer output capacitor plates resulting from the input signal and the resistive coupling thereby sensing the content.

A need nevertheless exists for improved monitoring of liquid and/or stool in a cost effective manner and with limited risk of false positives, and yet allow for accurate urine detection to not be compromised. There is further a need for novel monitoring systems that allow for reduced reliance on galvanic contact between all sensing components for improved flexibility and cleaning as well as minimizing article production costs; yet with improved sensing properties.

### SUMMARY

In a first aspect the disclosure relates to an absorbent article for personal hygiene comprising: a topsheet; a backsheet, wherein the topsheet is liquid permeable and the backsheet is substantially liquid impermeable; an absorbent core positioned between said topsheet and backsheet; a first indicator for indicating the presence of exudates and being positioned on a garment-facing side of said backsheet such that said first indicator is sandwiched between said backsheet and an outer cover of said article; or wherein said first indicator is positioned on a body-facing side of said backsheet such that said backsheet and an outer cover are able to come into contact with one another; and wherein said first indicator comprises an electrically conductive material, wherein the first indicator comprises one or more first input capacitors, one or more first output capacitors and a plurality of conductive tracks forming an electrical connection between the input and output capacitors, preferably such that each first output capacitor is in galvanic contact with each said first input capacitor. Wherein a reusable or single-use second indicator is arranged to be capacitively coupled with at least the first input and output capacitors such that upon a voiding event an amount of exudate may be automatically determined by measuring a dissipation factor or attenuation of an input signal such as a voltage, said second indicator comprising one or more second input capacitors, one or more second output capacitors and a sensing circuitry, and wherein the backsheet is breathable and has a water vapour transmission rate (WVTR) of at least 3000g/(m$^2$.24h), according to ASTM E96 A (Desiccant method); and/or wherein the outer cover (OC) is a nonwoven comprising synthetic fibers.

In a second aspect the disclosure relates to a system for sensing exudates comprising: the absorbent article and an external sensing unit, wherein said external sensing unit comprises the second indicator; and wherein said external sensing unit is releasably coupled with the absorbent article.

In a third aspect the disclosure relates to a kit comprising a plurality of absorbent articles and a detection device comprising the second indicator and operable with any one of said absorbent articles.

**BRIEF DESCRIPTION OF THE FIGURES**

FIG. 1 is a schematic top view of an absorbent article according to an embodiment of the present disclosure in the form of a diaper.

FIG. 2 is a schematic bottom view of an absorbent article according to an embodiment of the present disclosure in the form of a diaper.

FIG. 3 is a schematic top view of an absorbent article according to an embodiment of the present disclosure in the form of a diaper but showing a second indicator positioned on the garment facing side of the diaper being viewable.

FIG. 4 is a schematic cross-sectional view of an exemplary capacitor.

FIG. 5 is a schematic planar view of a second indicator according to an embodiment herein.

FIG. 6 is a schematic bottom view of an absorbent article according to an embodiment of the present disclosure in the form of a diaper.

FIG. 7 is a schematic bottom view of an absorbent article according to an embodiment of the present disclosure in the form of a diaper.

FIG. 8 is a schematic cross-sectional view of an absorbent article according to embodiments of the present disclosure such that of Fig.6-7.

FIG. 9 is a schematic system diagram according to an embodiment herein.

FIG. 10 is a schematic circuitry diagram according to an embodiment herein.

**DETAILED DESCRIPTION**

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed disclosure. However, it is to be understood that the value to which the modifier "about" refers is itself also specifically disclosed.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

As used herein, the "skin facing", "body-facing" or "bodyside" surface means that surface of the article or component which is intended to be disposed toward or placed adjacent to the body of the wearer during ordinary use, while the "outward", "outward-facing" or "garment-side" or "garment facing" surface is on the opposite side, and is intended to be disposed to face away from the wearer's body during ordinary use. Such outward surface may be arranged to face toward or placed adjacent to the wearer's garments or undergarments when the absorbent article is worn.

As used herein, the term "absorbent article" refers to disposable devices such as infant or adult diapers or pads, pants, training pants, and the like which are placed against or in proximity to the body of the wearer to absorb and contain the various exudates discharged from the body. Typically these articles comprise a topsheet, backsheet, an absorbent core and optionally an acquisition system (which may be comprised of one or several layers) and typically other components, with the absorbent core normally placed between the backsheet and the acquisition system or topsheet.

A "nonwoven web" as used herein means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yarn). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The terms "joined" or "bonded" or "attached", as used herein, encompasses configurations whereby an element is directly secured to another element by affixing the element directly to the other element, and configurations whereby an element is indirectly secured to another element by affixing the element to intermediate member(s) which in turn are affixed to the other element. The terms further include embodiments in which a pocket or other connector is formed in or attached to an area of the absorbent article. Further, these terms include configurations in which the elements are removably, or non-removably, joined, bonded, or attached. For example, wherein an element is described as "joined" within the configuration, it may be either removably joined or non-removably joined unless otherwise specified or evident from the context.

The terms "comprise," "comprising," and "comprises" are open ended terms, each specifies the presence of what follows, e.g., a component, but does not preclude the presence of other features, e.g., elements, steps, components known in the art, or disclosed herein. These terms based on the verb "comprise" should be read as encompassing the narrower terms "consisting of" which excludes any element, step, or ingredient not specified and "consisting essentially of" which limits the scope of an element to the specified materials or steps and those that do not materially affect the way the element performs its function. Any preferred or exemplary embodiments described below are not limiting the scope of the claims, unless specifically indicated to do so. The words "typically", "normally", "advantageously" and the likes also qualify elements which are not intended to limit the scope of the claims unless specifically indicated to do so.

By "absorbent material" it is meant a material which has some absorbency property or liquid retaining properties, such as SAP, cellulosic fibers as well as synthetic fibers, most preferably is selected from the group consisting of SAP, cellulose (or cellulosic) fibers, and mixtures thereof. Herein, absorbent materials in the form of fibrous absorbent materials have been found to be useful. These fibrous absorbent materials can comprise or consist of natural fibers, e.g. cellulosic fibers as well as synthetic fibers. Typically, glues used in making absorbent cores have no absorbency properties and are not considered as absorbent material.

As used herein, the term "absorbent core" refers to the component or components of the article having the most absorbent capacity and comprising an absorbent material and optionally a core wrap enclosing the absorbent material. The term "absorbent core" does not include the acquisition-distribution system or layer or any other component of the article which is not either integral part of the core wrap or placed within the core wrap. The core may consist essentially of, or consist of, a core wrap, absorbent material as defined below and glue enclosed within the core wrap.

The values indicated herein are measured according to the methods indicated herein below, unless specified otherwise.

The absorbent articles of the disclosure will be further illustrated in the below description and in the Figures in the form of a taped diaper, though all embodiments described herein may equally be applied onto absorbent articles in the form of pants (or even in the form of feminine hygiene or incontinence liners). Nothing in this description should be however considered limiting the scope of the claims unless explicitly indicated otherwise. Unless indicated otherwise, the description refers to the dry article, i.e. before use and conditioned at least 24 hours at 21° C.+/-2° C. and 50+/-20% Relative Humidity (RH).

THE ABSORBENT ARTICLE

Absorbent articles herein are generally for use in personal hygiene and may be selected from the group consisting of diapers, pants, incontinence inserts or briefs, and liners. Preferably such articles are adult incontinence absorbent articles.

Articles herein comprise a topsheet (TS); a backsheet (BS), wherein the topsheet is liquid permeable and the backsheet is substantially liquid impermeable; and an absorbent core (2) positioned between said topsheet and backsheet. The article further comprises an outer cover (OC) generally positioned on a garment facing side (or surface) of the backsheet. Optionally an acquisition and distribution layer (ADL) may be further comprised and positioned between the topsheet and the absorbent core. Further components may be comprised as known in the art such as lateral and/or transversal cuffs, elastic side panels, fastening systems and the like.

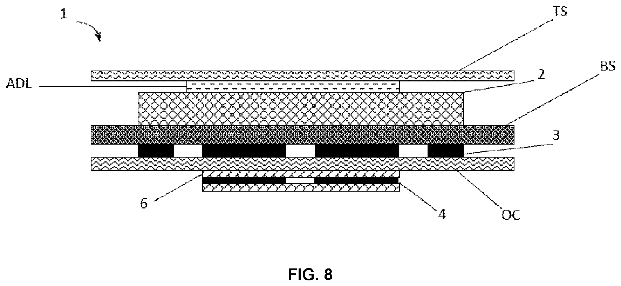

The absorbent article comprises a first indicator (3) for indicating the presence of exudates and being positioned on a garment-facing side of said backsheet such that said first indicator is sandwiched between said backsheet (BS) and an outer cover (OC) of said article (1), as for example illustrated in Fig. 8.

Alternatively, or additionally, the first indicator (3) is positioned on a body-facing side of said backsheet such that said backsheet and an outer cover (OC) are able to come into contact with one another. The outer cover (OC) may be brought into contact with the backsheet (BS) by one or more joining means such as adhesive. When adhesive is used, it may be applied in the form of a spray or slot-coat such to be substantially uniformly applied at least in the region of overlap between first indicator (3) and second indicator (4) as explained herein. Advantageously this permits a more uniform and predictable dielectric on which measurements of attenuation and/or dissipation may be based, it also allows the layers to be in close contact with one another and limit the risk of formation of wrinkles or other pleat-like formations that may impact uniform thickness and hence be a source for error generation in the measurements.

The first indicator (3) comprises an electrically conductive material, and comprises one or more first input capacitors (3i), one or more first output capacitors (3ii), and a plurality of conductive tracks (3iii) forming an electrical connection between the input and output capacitors, typically such that each first output capacitor (3ii) is in galvanic contact with each said first input capacitor (3i). The conductive tracks (3iii) typically comprise one or more electrodes that are spaced apart such that electrical connection between the input and output capacitors occurs and/or increases (e.g. a resistance is reduced) when an exudate (and/or liquid and/or humidity) bridge is formed between said electrodes. Advantageously such galvanic coupling allows to monitor changes in one or more electric properties in one or more zones of the absorbent article upon a voiding event.

A reusable or single-use second indicator (4) is further arranged to be capacitively coupled with at least the first input and output capacitors such that upon a voiding event an amount of exudate may be automatically determined by measuring a dissipation factor or attenuation of an input signal, such as a voltage or current signal. Advantageously the capacitive coupling between the first and second indicators allows to dispense more complex or costly solutions that may be required to enable direct electrical contact and hence risk of damage to the backsheet of the article may in addition be further reduced.

The second indicator (4) comprises one or more second input capacitors (4i), one or more second output capacitors (4ii) and a sensing circuitry (SC). Advantageously, first and second indicators may cooperate synergistically to combine the benefits of reliability of resistance changes upon exudate (and/or liquid and/or humidity) contact within tracks of the first indicator (not only for measuring an amount of liquid but further allowing also position and/or location to be further monitored) and further, rather than direct resistance measurements, usage of the capacitive connection with the second indicator to measure a dissipation factor (or power loss or voltage attenuation) through the respective circuit formed by the first and second indicators together.

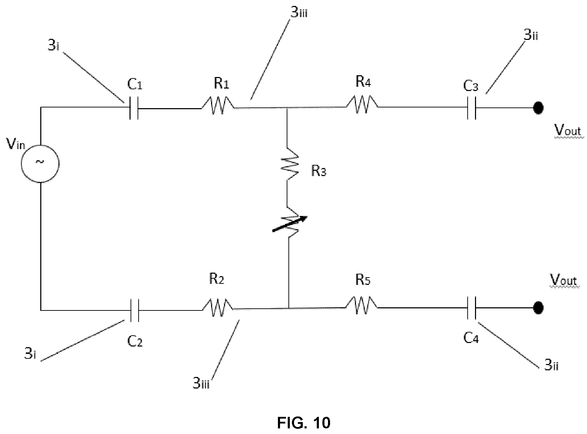

The sensing circuitry may be further arranged to apply an input signal onto the second input capacitor plate(s), and measure an output signal from the second output capacitor plate(s) resulting from the input signal and the resistive coupling thereby sensing the diaper content. The first and second indicators may form a circuitry as exemplarily illustrated in Fig. 10 and that will be explained in more detail herein.

The absorbent articles herein are further characterised by comprising a backsheet (BS) that is breathable and has a water vapour transmission rate (WVTR) of at least 3000 g/(m$^2$.24h), preferably at least 4000 g/(m$^2$.24h), according to the method ASTM E96 A (Desiccant method). Preferably, the water vapour transmission rate (WVTR) is from 4500 g/(m$^2$.24h) to 10000 g/(m$^2$.24h), preferably from 5000 g/(m$^2$.24h) to 9000 g/(m$^2$.24h), even more preferably from 5500 g/(m$^2$.24h) to 8500 g/(m$^2$.24h), even more preferably from 6000 g/(m$^2$.24h) to 8200 g/(m$^2$.24h), according to the method herein. Breathability of the backsheet is not only advantageous in reducing skin irritation but surprisingly it has been further found that a sufficiently high breathability aids better exudate detection in systems herein as it allows liquid (generally in the form of water vapor) to permeate through the backsheet and hence better trigger a change in resistance of the electrically conductive material of the first indicator that is typically printed on the backsheet of the article. Moreover, this arrangement allows to no longer require the conductive material of the first indicator to be e.g. printed on a skin (or body)-facing surface (or side) of the backsheet but rather permits its location on a garment-facing surface (or side) thereof. The ranges herein have been found particularly suitable for ensuring sufficient permeation of humidity for accurate electrical measurements as described herein and yet ensure no excessive wetting of the garments of a wearer occurs, to hence provide a sweetspot for optimal operation.

In addition, or alternatively, the outer cover (OC) is a nonwoven comprising synthetic fibers. Advantageously such nonwovens are not only advantageous in providing a soft-to-the-touch sensation for reduced skin irritation for subjects and care givers handling the articles, but importantly it has been surprisingly found that such materials aid better exudate detection in systems herein as it allows the formation of a good dielectric for capacitor couplings herein.

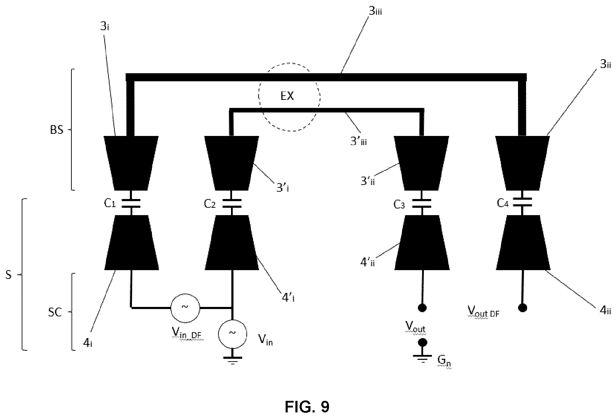

As exemplarily illustrated in Fig. 9-10, the circuitry layer formed by the first indicator (3), e.g. as printed onto the backsheet with a conductive ink as explained in embodiments herein, is part of the absorbent article and provides the actual content sensing by means of the arranged tracks. The input signal is applied to these tracks and transformed to the output signal by the resistive coupling that is dependent on the exudate content within the article, e.g. the extent of an attenuation of the input signal is dependent on the exudate content within the absorbent article. Input and output signals are coupled between the first indicator (3) and second indicator (4) by a capacitive coupling. The coupling capacitors are formed by the first capacitor plates within the absorbent article and by the second capacitor plates of the re-usable sensor (s) that is typically coupled to the outer cover (OC) of the article as described in embodiments herein, with layers therebetween serving as dielectric. Generally, as the input signal is applied to the input capacitors and the output signal is measured from the output capacitors, the resulting measurement may be a 4-port transmission measurement describing how the input signal is transformed by the transmission path to the output signal. This transmission path is formed by the resistance of the inner capacitor plates, by the resistance of the tracks and by the resistive coupling between the tracks dependent on the amount of exudates present in the absorbent article. It is an advantage that the sensing of exudate content within the article by the circuitry layer formed by the first indicator (3) is obtained by a passive circuitry as both the tracks and capacitor plates are passive electrical components. There is thus no need to provide active components within the article. It is a further advantage that there is no galvanic connection needed between the circuitry layer formed by the first indicator (3) and the coupling layer formed by the second indicator (4) thereby avoiding the need for making holes or pockets through the article or any other means to achieve direct connection. Further, by the capacitive coupling and resistive sensing, the measurements do not require large currents provided some care is taken in the choice of backsheet and outer cover as explained herein. As a result, the circuitry layer formed by the first indicator (3) does not require high conductance for the tracks and capacitor plates. Therefore, the tracks and first capacitor plates may be made of materials having a lowconductance, e.g. non-metal based conductive materials, while still achieving a good content sensing.

In the embodiments herein, the input signal may comprise a differential input signal. The sensing circuitry may then arranged to apply the differential input signal between two second input capacitor plates and to measure a differential output signal from two correspondingly connected second output capacitor plates. In such case there are at least two input capacitors and two output capacitors formed by the first and second indicators. By the use of differential signaling, external disturbances can be minimized resulting in a better content detection. The change in exudate content may then be detected by measuring a change in the differential output signal. Further, the input signal may also comprise a common mode input signal. The sensing circuitry may then be arranged to apply the common mode input signal to the two second input capacitor plates, and to measure a common mode output signal from the two correspondingly connected second output capacitor plates, and to compensate the measured diaper content for a change in electrical coupling between the input -and output capacitors based on the common mode output signal.

In an embodiment, a first first input capacitor plate of the first input capacitor plates and a first first output capacitor plate of the first output capacitor plates are provided at distal ends of a first track of the tracks and wherein a second first input capacitor plate of the first input capacitor plates and a second first output capacitor plate of the first output capacitor plates is provided at distal ends of a second track of the tracks thereby defining a detection zone for sensing the exudate content within the article and between the first and second track. Advantageously, this topology creates a relatively long distance between the input and output plates because the track is located in between them. The resistive coupling is then provided between the different tracks along this long distance, i.e. the tracks are arranged from inputs to outputs in a series configuration. By this topology very small quantities in exudate content can still be detected.

In an embodiment, a first first input capacitor plate of the first input capacitor plates and a first first output capacitor plate of the first output capacitor plates is provided at a same distal end of a first track of the tracks and wherein a second first input capacitor plate of the frist input capacitor plates and a second first output capacitor plate of the first output capacitor plates is provided at a same distal end of a second track of the tracks thereby defining a detection zone for sensing the diaper content between the first and second track. This topology creates a short distance between the input and output plates in comparison with the track itself thereby arranging the tracks in a shunt configuration. By this topology differences in exudate content can be detected when having high moisture levels such as large amounts of urine being present.

Fig. 9-10 illustrate a system for sensing exudate content (EX) within an absorbent article generally making its way to the backsheet thereof containing the first indicator (3). The system generally comprises an absorbent article having a backsheet (BS), a coupling layer formed by a re-usable sensor (S) and a sensing circuitry (SC). The backsheet may be provided with an first circuitry layer corresponding to the first indicator (3). The first circuitry layer may comprise input capacitor plates (3i, 3'i), conductive tracks (3iii, 3'iii) and output capacitor plates (3ii, 3'ii). A first input capacitor plate (3i) may be connected by a first conductive track (3iii) with a first output capacitor plate (3ii) thereby galvanically connecting capacitor plate (3i) with capacitor plate (3ii). Similarly, a second input capacitor plate (3'i) may be connected by a second conductive track (3'iii) with a second output capacitor plate (3'ii). Input capacitor plates (3i, 3'i) may thereby be in galvanic contact with the respective output capacitor plates (3ii, 3'ii). The conductive tracks (3iii, 3'iii) may define a detection area or zone within the backsheet (BS) of the absorbent article. When there is an exudate (EX) discharge within this detection zone, as illustrated by the dashed circle (EX) , then the resistance between the first and second track (3iii, 3'iii) will decrease e.g. due to the moisture thereof. As such, a transmission path may be formed which will transform input signals to the output signals. This transmission path may then be formed from input to output by the serial resistor formed by a plurality of resistors in series and by the variable parallel resistor formed by the variable resistor (see e.g. Fig.10).

The system generally further comprises re-usable sensor (S) further comprising input coupling capacitor plates (4i, 4'i) and output coupling capacitor plates (4ii, 4'ii). The coupling capacitor plates (4i, 4'i, 4ii, 4'ii) may be arranged on the coupling layer of the re-usable sensor (S) such that they form respective capacitors with the respective capacitor plates (3i, 3'i, 3ii, 3'ii) as provided on the backsheet (BS). As a result, input capacitor plates (4i, 4'i) may allow receiving an input electrical signal and coupling the electrical signal into the first circuitry layer or first indiactor (3). Similarly, output capacitor plates (4ii, 4'ii) may allow coupling out an electrical signal from the first circuitry layer (otherwise referred to as first indicator (3)) as output electrical signals.

The system may further comprise a sensing circuitry (SC). Sensing circuitry (SC) may be arranged to apply a differential input signal between input coupling capacitor plates (4i, 4'i), e.g. a signal characterised by the voltage difference that is applied between the said plates (4i, 4'i). Sensing circuitry (SC) may further be arranged to apply a common mode signal to both input coupling capacitor plates (4i, 4'i), e.g. a signal characterised by the voltage difference between a common ground node and one of the input coupling plates (4i, 4'i). Input signals, whichever of the kind described above, will be coupled into the backsheet (BS) of the absorbent article and/or the article itself by capacitors (C1, C2) and appear as voltages on the input capacitor plates (3i, 3'i). Dependent on the moisture content within detection zone, the applied input signals will generally cause electrical output voltages on the output capacitor plates (3ii, 3'ii). By coupling output capacitors (C3, C4), the output voltages are coupled out of the backsheet (BS) onto the output coupling capacitor plates (4ii, 4'ii). Sensing circuitry (SC) is then able to measure the differential output signal as the electrical voltage difference between the plates 4ii and 4'ii. Sensing circuitry (SC) may further measure the common mode output signal as the electrical voltage difference between a common ground node (Gn) and one of the output coupling plates 4ii, 4'ii.

As explained herein-above, the second indicator (4) may be in the form of a re-usable sensor (S) adapted to be joined to the outer cover (OC) of the absorbent article by means of a releasable coupling. Preferably, the re-usable sensor (S) is in the form of a coupling strip that is releasably joinable to the outer cover. For example, the outer cover (OC) may comprise an adhesive (typically in the form of one or more stripes of said adhesive) and covered with a release paper. When it is desired to join the coupling strip to the outer cover, the release paper may be removed to directly expose the underlying adhesive and said coupling strip is placed thereon to secure said coupling strip to the outer cover (OC).

Preferably, the second indicator (4) is in electrical communication with a power source providing an alternating current (AC) and at least in data or signal communication with a processing unit. Alternating current is advantageous for attenuation or dissipation measurements as will be described in more detail hereinbelow.

The conductive tracks are generally arranged to undergo a change in one or more electric properties in response to a voiding event such as direct or indirect contact with exudates and/or moisture caused therefrom.

Preferably the outer cover (OC) is a nonwoven comprising, preferably consisting of, synthetic fibers and wherein said fibers comprise, preferably consist of, polyprolylene; and/or wherein the outer cover (OC) is a nonwoven having a basis weight of from 5gsm to 25gsm, preferably from 6gsm to 20gsm, even more preferably from 7gsm to 18gsm. Having an appropriate dielectric material between the capacitor plates allows for accurate measurement of attenuation or dissipation factor as described herein, yet, it has been found that equally important is that the capacitor plates are as close as possible to limit errors or noise in the detection data caused by for example electrical field disturbances. Polypropylene in particular has been found to be an excellent dielectric material compared to for example other polymers for use in absorbent articles such as polyethylene or polylactic acid, and hence permitting to reduce the amount needed thereof. Moreover, a low basis weight nonwoven in the ranges provided above has been found particularly suitable to maintain indeed sufficient separation of the capacitor plates whilst not being too high to cause the undesirable filed disturbances described. An outer cover as described is particularly useful when the backsheet is a film comprising, or consisting, of polyethylene.

The outer cover (OC) is preferably a spunbond nonwoven or a laminate comprising one or more spunbond nonwoven layers. Example of said laminates comprise a combination of spunbond S layer(s) and meltblown M layer(s) and include laminates such as spunbond-meltblown (SM), spunbond-meltblown-spunbond (SMS), spunbond-meltblown-meltblown-spunbond (SMMS), spunbond-spunbond-meltblown-spunbond (SSMS), laminates and the like. Compared to other nonwovens that may have other desirable attributes such as carded nonwovens that are more and more used as outer covers for absorbent articles, spunbond nonwovens and laminates thereof have been found to not only advantageously limit bulkiness and thickness but moreover the more compacted nature of the fibers limits the amount of void spaces therebetween hence resulting in an excellent dielectric material for the purposes of the present disclosure whilst retaining some functionality such as softness to the touch for caregivers and subjects using the article.

The first indicator (3) may be printed with a conductive ink, preferably wherein the conductive ink is a carbon based ink and/or a conductive polymer based ink. Advantageously, this allows the first indicator to be printed over a larger area of the backsheet such that different sensing zones are formed planarly along and across the absorbent article whilst limiting the amount by volume of conductive material used.

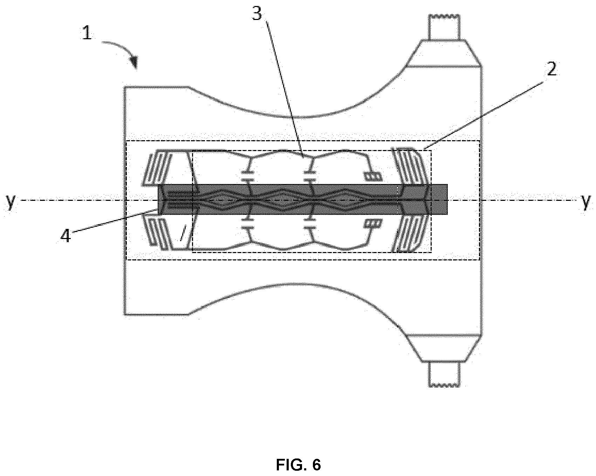

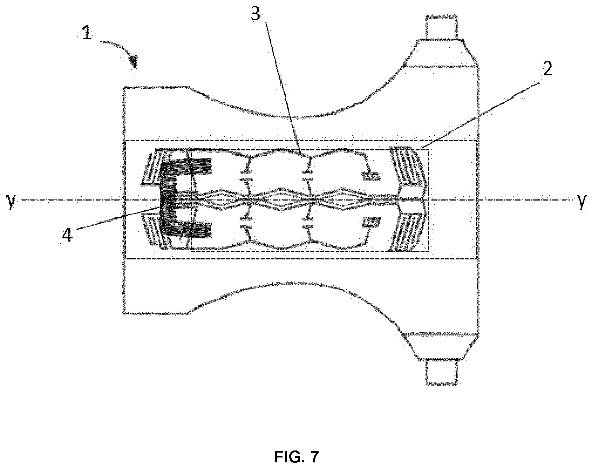

As for example illustrated in Fig. 6-7, a total surface area of the first indicator (3) may be greater than a total surface area of the second indicator (4), wherein said surface area is taken about a plane corresponding to the backsheet (BS), preferably wherein the total surface area of the first indicator (3) is more than 2 times, preferably more than 3 times, even more preferably from 4 to 10 times, the total surface area of the second indicator (4). Advantageously a larger coverage of the backsheet by the first indicator maximises exudate detection accuracy, especially with the ability of sensing the presence of exudates at different positions of the absorbent article, yet concentrating the second indicaror (4) to overlap only selected areas of the first indicator (3) generally corresponding with capacitor plates to be overlapped, permits to limit material usage and power requirements of the device for a more sustainable and longer lasting product.

The first and second indicators (3, 4) may have different shapes, preferably wherein the first indicator comprises a first shape and the second indicator comprises a second shape and wherein the second shape overlaps a portion of the first shape with portions of the first shape extending inboard and/or outboard of the second shape. More preferably, the overlapped portion is less than 30%, preferably from 2% to 25%, even more preferably from 5% to 20%, of the total surface area of the first indicator (3). Advantageously this permits further improvement in material usage and power consumption as described above.

In an embodiment, the second indicator (4) is coated and/or contained within a dielectric sleeve (6), preferably said coating and/or sleeve comprises silicone. Advantageously, this allows for easy cleaning and maintenance of the removable sensor coupling strip and yet at the same time further acts as a dielectric material summing to the dielectric constant used by the device when measuring attenuation or dissipation factor.

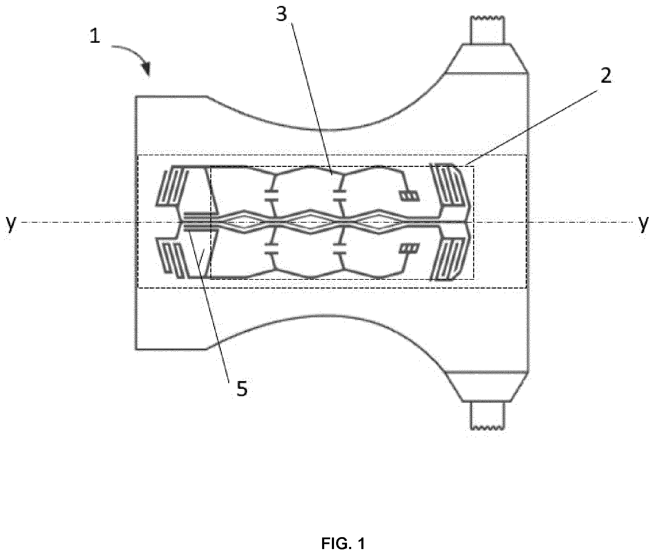

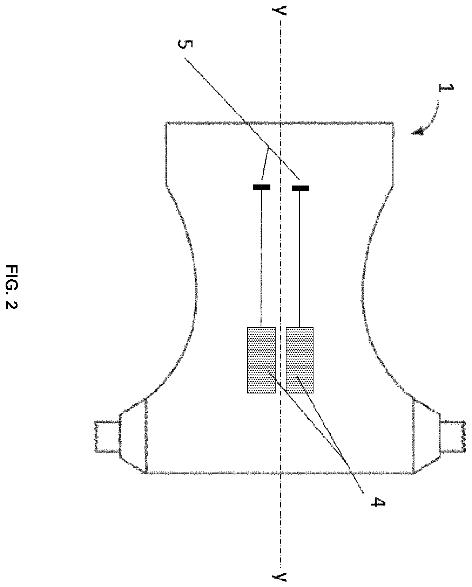

As exemplified in Fig.1 to Fig.3, absorbent articles herein may be for personal hygiene such as a diaper, or pant, or incontinence insert or pad or liner, the absorbent article may comprise: a topsheet; a backsheet, wherein the topsheet is liquid permeable and the backsheet is substantially liquid impermeable; an absorbent core (2) positioned between said topsheet and backsheet; a first indicator (3) for indicating the presence of exudates and being positioned on a body-facing side of said backsheet, said first indicator (3) comprising an electrically conductive material, wherein said article (1) may comprise a second indicator (4) for indicating the presence of exudates and being positioned on a garment-facing side of said backsheet such that the first and second indicators (3, 4) are separated from each other at least in a thickness direction (i.e. a direction substantially following a z-axis that crosses the topsheet, absorbent core, and backsheet; and that may be substantially perpendicular to the longitudinal axis), said second indicator (4) may comprise an electrically conductive material, and wherein said first indicator (3) may comprise a first application pattern and said second indicator (4) may comprise a second application pattern, wherein said first and second application patterns are different. Advantageously, this arrangement allows for accurate automated urine detection by the first indicator and stool detection by the second indicator whilst minimising interference effects in readings which may otherwise result in undesirable false positives being triggered.

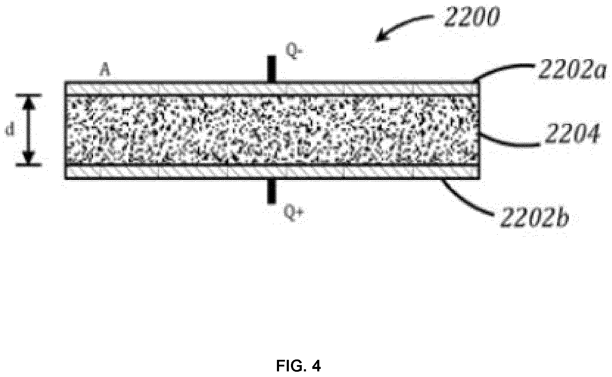

Fig.4 shows a cross-sectional view of an exemplary capacitor 2200. Capacitor 2200 includes two metal conductive plates 2202a and 2202b, which are separated by a dielectric material 2204.

The capacitance C is given by:

$$C = \frac{\varepsilon_0 \varepsilon_r A}{d},$$

where:

A is the area of overlap between the two plates;

$\varepsilon_r$ is the relative static permittivity (also known as the dielectric constant) of the material between the plates (for a vacuum, $\varepsilon_r = 1$);

$\varepsilon_0$ is the electric constant ($\varepsilon_0 \approx 8.854 \times 10\text{-}12$ F m-1); and

d is the distance between the plates.

Urine and stool may be considered as having the characteristics of an electrolyte solution. Hence, they may influence the electrical field in their surroundings. The second indicator (4) and the secretion/exudate (e.g. stool) serve here as the 2202b and 2202a plates of capacitor 2200 of Fig.4 accordingly. The backsheet (and/or outer cover) with internal diaper absorption material such as the absorbent core serves here as dielectric layer 2204 of capacitor 2200 of Fig.4. Such configuration implements the physics of a capacitor and may be referred to as a diaper capacitor. Thus, the diaper capacitor may be connected in series with capacitive element of detector.

In order to detect the capacitance in steady state and during change, the detector may be coupled with suitable electronics. The electronics are either embedded in the diaper during the manufacturing process, or externally coupled with the diaper, e.g. with the outer shell of the diaper and/or applied thereto by other means such as printing and the like.

Since secretion appears to be a part of the capacitor of the absorbent article (e.g. a diaper), it may immediately influence the capacitance magnitude of capacitive detector.

In an embodiment, a photodetector is used as a sensor for detecting urine secretion and/or stool secretion. As urine and/or stool are secreted into the diaper, the amount of light reaching a photodetector decreases, since a greater portion of the light may now be blocked by the secretions. Generally, the amount of light reaching the photodetector changes as a function of the amount of urine and/or stool disposed inside the diaper. An LDR sensor may be based on the principle of a decreasing resistance when light incidence increases.

A circuit may comprise an LDR detector and/or a capacitor connected to operational amplifier converter/conditioner. The operational amplifier may have high input impedance and unity gain, and if LDR is used, the principle may be based on a voltage divider between a fixed resistor, referred also as $R_m$, and LDR, referred also as $R_{photo}$. The output voltage $V_{out}$ may be given by:

$$V_{out} = \frac{V_{cc}}{(1 + R_m/R_{photo})},$$

i.e. output voltage is rather linear to LDR resistance. Alternatively, an integrator circuit including capacitive sensor, resistor and amplifier may be used (instead of resistor and LDR). The value of the output voltage (indicated $V_{out}$) depends on the value of capacitive sensor charge or discharge as a function of time.

An LDR and a 2MΩ resistor may serve as a voltage divider. When light level is low (in our case, when pieces are interlocked), the resistance of LDR may be high. This may prevent current from flowing to the base of the transistor. Consequently, the output voltage may be low, commonly close to 0 volts. However, when light illuminates the LDR without much interference (e.g. in case, when pieces are not interlocked) the resistance may fall and current may flow into the base of transistor, increasing the output voltage to high level (e.g. about 5 volts). Alternatively, an integrator circuit including capacitive sensor, resistor and amplifier may be used (instead of resistor and LDR).

The absorbent article may comprises one or more indicator(s) adapted to indicate the presence and/or absence of bodily exudates and/or determine saturation level and when the article should be replaced with a new clean one. The indicator, in some embodiments, for example, may comprise an indicator that reacts to the presence and/or absence of bodily exudate(s) and/or one or more properties of those bodily exudate(s) within the absorbent article via one or more change in property of the indicator (e.g., a physical, chemical or biological property such as color, smell, sound, pH, or the like). One or more property or state of the indicator, in turn, may be detected by a detector device physically and/or communicatively coupled to the absorbent article. In one particular implementation, for example, the indicator comprises an optical property changing composition or device (e.g., a color-changing composition or device, such as a color changing indicator) that changes an optical property (e.g., color) in response to a variation of pH associated with the presence and/or absence of bodily exudates within the absorbent article). The indicator might also comprise one or more additional indicators of the same or different type that provide different types of indications and/or indications of bodily exudates (or properties of bodily exudates) detected in one or more different regions of the absorbent article. In one embodiment, for example, a second electrical indicator may comprise a resistance, capacitance, inductance or continuity sensitive indicator. Alternatively, such electrical indicators may be provided as an alternative or in conjunction with optical indicators. A resistance sensitive indicator can be provided, for example, by providing two or more electrical conductors disposed at a given spatial distance relative to each other. If bodily exudates, which typically comprise a liquid portion, come in contact with the two electrical conductors, the resistance between the two electrical conductors is reduced (examples of this arrangement are described in more detail in applicant's prior disclosures such as EP3415130, WO/2018/228822, WO/2018/229017, EP3461257, and EP3451988). Other indicators, as known in the field in the context for sensor for absorbent articles, can also be useful. In one particular embodiment, for example, the multiple property changing indicators may be provided in the same or different locations within the absorbent article. For example, an optical property changing indicator (e.g., color changing indicator) may be disposed in a first location of an absorbent article and a second property changing indicator that is the same or a different type of indicator (e.g., another optical property changing indicator such as a color changing indicator) may be disposed in a second location of the absorbent article.

The absorbent article and the one or more indicators may be provided to form an integral unit. For forming the integral unit, the indicator(s) can be directly or indirectly attached to the absorbent article. Direct or indirect attachment to the article is typically to one or more distinguishable element of the article. For example, it can be useful to attach the indicator(s) to the back sheet of the article, such that the indicator(s) and the back sheet of the article from one integral unit. For example if the indicator(s) are provided in sheet form, the respective sheet can be adhesively attached to the back sheet of the article. The respective sheet could also be provided from one and the same material with the back sheet, this material however being treated in suitable ways as to provide an indicator in a pre-defined area (for example by printing an electrically conductive material on a skin or body facing side of the liquid impermeable backsheet layer).

According to one particular embodiment, a detector device (herein also referred to a clip-on processing unit or clip-on unit) is also provided. The detector device, in this implementation, comprises a housing and is adapted to be physically coupled to the absorbent article such that the detector device is further communicatively coupled to one or more indicator integral with the absorbent article and/or the removable coupling strip described hereinabove. The detector device and/or the absorbent article may comprise one or more connector for removably joining the detector device with the absorbent article. The connector(s) are provided such that the detector device can be attached to the absorbent article and can be detached from the absorbent article including the one or more indicator(s). The detector device can be attached to the integral unit and can be detached from the integral unit. In one particular embodiment, for example, the detector device can be attached to an area of the absorbent article juxtaposed the indicator integral to the absorbent article, and can be detached from that area of the absorbent article so as to be removably connectable thereto.

The housing of the detector device, in one embodiment, has an outer extension in a first direction and an outer extension in a second direction, which is perpendicular to the first direction. The first direction, in this embodiment, may be chosen as characteristic directions, e.g. along a main axis and normally as that of largest extension of the housing. For safety and convenient handling of the device, it may be useful that the device has a length in the first direction of at least 3 cm, 4 cm or more (but normally less than 10 cm) and that the device has a length in the second direction of at least 2 cm, 3 cm or more (but normally less than 10 cm). In one particular embodiment, for example, the housing has a first dimension of at least about 4cm and a second dimension of at least about 8cm. In various embodiments, the housing can be rigid or at least partially or fully flexible. To be flexible the detector device can incorporate flexible electronic components (and boards).

According to one embodiment, the detector device comprises one or more optical sensor, such as a color sensor. This optical sensor can generate an output which depends on an optical property (e.g., a color) observed by the optical sensor. Some examples of optical sensors across a range of wavelengths are: electron tube detectors, photosensors, photomultiplier tubes, phototubes, photodetectors, opto-semiconductor detectors, photodiodes, photomultipliers, image sensors, infrared detectors, thermal sensors, illuminance sensors, visible light sensors and color sensors. In one particular embodiment, for example, the optical sensor may comprise a photodiode such as a TCS 34725 color sensor commercially available from AMS-TAOS USA Inc.

In other embodiments, for example, the detector device need not include a light source, such as where sufficient ambient light may be provided in an application, where light is provided elsewhere (e.g., associated with an absorbent article or clothing, or elsewhere in an environment) or where the property change of a property changing indicator may be detectable without light, such as resistance, impedance and/or capacitance measurements when the indicator comprises an electrically conductive material (such as a conductive ink).

Often, but not necessarily, the detector device will also comprise one or more light, such as a light emitting diode (LED), organic light emitting diode (OLED), an incandescent light bulb, thermionic light emission, luminescence (e.g., among others, fluorescence, chemilluminescence, electroluminescence (e.g., LED), for emitting light onto an area, the wavelength or spectrum of which is to be assessed by the optical sensor. The optical sensor in some color detecting embodiments can be optimized for assessing a color of a color-changing indicator. The optical sensor can be sensitive to visible and non-visible light, namely light in the near IR range. In various embodiments, UV, visible infrared and near infrared wavelengths may be used. A color changing indicator can change its color, for example, based on the presence and/or absence of bodily exudates and/or in response to some other condition being monitored with respect to the absorbent article. In this embodiment, the color sensor can provide an output that varies depending on the presence or absence of bodily exudates.

In a preferred embodiment, the absorbent article further comprises a detection device that can be removably attached to the absorbent article such to become in electrical communication with the first and/or second indicators (3, 4) when attached to said article to measure a change in resistance and/or capacitance in response to a voiding event. A single such detection device connecting to all indicators or one detection device for each of the indicators may be used. Suitable detection devices for use herein are for example described in EP3906907 A1.

Preferably, the first application pattern is applied over a first area of the backsheet and the second application pattern is applied over a second area of the backsheet, and wherein the first area is greater than the second area, preferably wherein the first area extends up to at least proximal to and/or adjacent one or more perimeter edges of the absorbent core (2), and typically even extends beyond said perimeter edges, and the second area is inboard and distal from one or more perimeter edges of the absorbent core (2) such that it is proximal to and/or adjacent and/or overlaps with at least a portion of the longitudinal axis (y) extending substantially parallel to a longest dimension of said article (1). The second area may fall within and/or be positioned inboard of the first area. Preferably wherein the second area is located, typically only, at a back portion of the absorbent article. The back portion typically being delimited by a transversal centreline of the absorbent article (being perpendicular to the longitudinal axis y) and a back transversal edge of the article opposite and substantially parallel thereto.

In an embodiment, the pattern of the first indicator (3) comprises a plurality of sensing tracks, preferably wherein each track forms an open and/or closed circuit corresponding to a different position of the backsheet and the absorbent core such that voiding events in said different positions may be individually monitored; and wherein the pattern of the second indicator (4) comprises one or more sensing tracks having a shape selected from the group consisting of linear, polygonal, and spiral-shaped. Preferably, each of the sensing tracks comprises a connection end (5) arranged to couple to a respective terminal end of the detection device to form an electrical connection between said detection device and said sensing tracks of the indicator. Advantageously this allows for wide coverage for the urine detection which may also permit more accurate warnings to be triggered in case of higher risk of leakage, and targeted detection of stool at about the insult position.

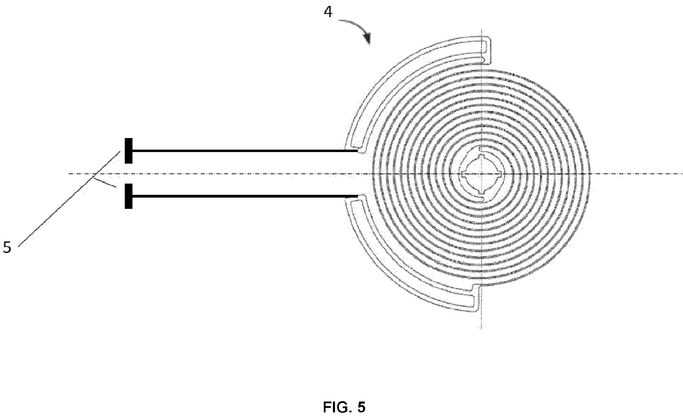

Fig. 5 exemplifies a spiral-shaped second indicator (4) that may be used in absorbent articles herein. Advantageously this arrangement allows to produce a sufficiently strong electric field for capacitance measurements whilst covering a larger surface area under an insult position yet using a lower total amount of conductive material.

In an embodiment, the first and/or second indicators (3, 4) are printed with a conductive ink, preferably wherein the conductive ink is a carbon-based ink and/or a conductive polymer-based ink. Preferably, the carbon-based ink comprises a conductive compound selected from the group consisting of graphene, graphite, nano-carbon-tubes and mixtures thereof. Preferably, the conductive polymer-based ink comprises a polymer selected from the group consisting of polyacetylene, polypyrrole, polyaniline and copolymers thereof, more preferably selected from the group consisting of poly(pyrrole)s (PPY), polyanilines (PANI), poly(thiophene)s (PT), poly(p-phenylene sulfide) (PPS), poly(p-phenylene) (PPP), Poly(acetylene)s (PAC), Poly(p-phenylene vinylene) (PPV), poly(3,4-ethylenedioxythiophene) (PEDOT), and mixtures thereof, most preferred conductive polymer-based ink comprising poly(3,4-ethylenedioxythiophene) polystyrene sulfonate (PEDOT:PSS).

In an embodiment, the indicators (3, 4) are free of metals.

In an embodiment, the absorbent article further comprises a color-changing wetness indicator. When such wetness indicator is present, the detection device may further comprise an optical sensor and one or more light sources that may be as described herein.

In an embodiment, the first indictor (3) is an electrically conductive sensor adapted to change one or more of its electrical properties in response to a voiding event such as presence of exudates, preferably urine, in the absorbent article. Preferably, the one or more electrical properties is resistance; or resistance combined with one or more of capacitance, inductance, and combinations thereof. Preferably, the first indicator (3) is adapted to detect each voiding event of a plurality of, typically sequential, voiding events throughout a wearing period of said absorbent article by a wearer.

In an embodiment, the second indicator (4) is an electrically conductive sensor adapted to change one or more of its electrical properties in response to a voiding event such as presence of exudates, preferably stool, in the absorbent article, most preferably wherein the one or more electrical properties is capacitance. Advantageously this works synergistically with the at least resistance measurement of the first indicator to help exclude false positives. For example a capacitance trigger by the second indicator combined with an absence of a resistive trigger by the first indicator is more likely to be a stool voiding event that should trigger a higher priority warning for a given patient to be changed.

In an embdoment, the backsheet is breathable and a dielectric coating is comprised on the body-facing or garment-facing side of the backsheet at positions corresponding to, preferably only, the second indicator (4) and/or the second application pattern such that one or more areas neighbouring said second indicator (4) and/or second application pattern are free of said coating. The dielectric coating may be in the form of a varnish or other resin-comprising coating. Advantageously this allows to insulate the surface over which the capacitance measurements are to be made for improved accuracy whilst limiting reduction in breathability of the surface which remains important for skin-care.

In an embodiment, the first indicator (3) and second indicator (4), and/or the first application pattern and the second application pattern, substantially do not overlap for a substantial portion thereof when viewed in a planar direction (e.g. from top or bottom views as shown in figures 1-3). Advantageously this may limit electric field interference that may impact accuracy.

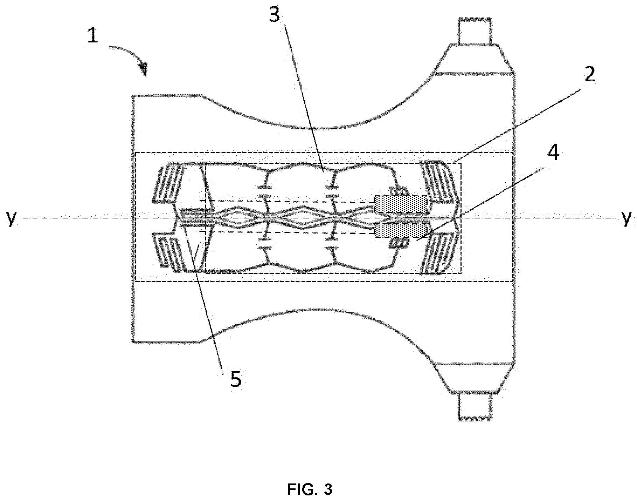

In a preferred embodiment, the first indicator (3) and second indicator (4), and/or the first application pattern and the second application pattern are substantially complementary generally such that when the first indicator (3) and second indicator (4), and/or the first application pattern and the second application pattern are overlayed they substantially fit one within the other. This may be as exemplified in Fig. 3. Advantageously this arrangement limits electric field interference that may impact accuracy as well as optimise surface coverage for exudate detection.

These and other components of the articles will now be discussed in more details.

The topsheet is generally the part of the absorbent article that is directly in contact with the wearer's skin. The topsheet can be joined to the backsheet, the core 2 and/or any other layers as is known in the art. Usually, the topsheet and the backsheet are joined directly to each other in some locations (e.g. on or close to the periphery of the article) and are indirectly joined together in other locations by directly joining them to one or more other elements of the diaper.

The topsheet is preferably compliant, soft-feeling, and non-irritating to the wearer's skin. Further, at least a portion of the topsheet is liquid permeable, permitting liquids to readily penetrate through its thickness. A suitable topsheet may be manufactured from a wide range of materials, such as porous foams, reticulated foams, apertured plastic films, or woven or nonwoven materials of natural fibers (e.g., wood or cotton fibers), synthetic fibers or filaments (e.g., polyester or polypropylene or bicomponent PE/PP fibers or mixtures thereof), or combinations thereof, e.g. a combination of natural and synthetic fibers. A combination of materials can be achieved by combining at least two materials by means of needle punching, ultra-sonic bonding, ring rolling, embossing, gluing or other types of mechanical entanglement. The resulting material may maintain a dual/-multiple layer structure, but may also loose a structure of distinguishable layers after such process steps. It can also be useful to provide a formed film patch underneath the topsheet.

If the topsheet includes fibers, the fibers may be spunbond, carded, wet-laid, meltblown, hydroentangled, or otherwise processed as is known in the art, in particular spunbond PP nonwoven. A suitable topsheet comprising a web of staple-length polypropylene fibers is manufactured by Veratec, Inc., a Division of International Paper Company, of Walpole, Mass. under the designation P-8.

The topsheet may comprise one or more apertures to ease penetration of exudates therethrough, such as urine and/or feces (solid, semi-solid, or liquid). The size of at least the primary aperture is important in achieving the desired waste encapsulation performance. If the primary aperture is too small, the waste may not pass through the aperture, either due to poor alignment of the waste source and the aperture location or due to fecal masses having a diameter greater than the aperture. If the aperture is too large, the area of skin that may be contaminated by "rewet" from the article is increased. Typically, the total area of the apertures at the surface of a diaper may have an area of between about 10 mm2 and about 50 mm2, in particular between about 15 mm2 and 35 mm2. Examples of apertured topsheet are disclosed in U.S. Pat. No. 6,632,504, assigned to BBANONWOVENS SIMPSONVILLE. WO2011/163582 also discloses suitable colored topsheet having a basis weight of from 12 to 18 gsm and comprising a plurality of bonded points. Each of the bonded points has a surface area of from 2 mm2 to 5 mm2 and the cumulated surface area of the plurality of bonded points is from 10 to 25% of the total surface area of the topsheet. Typical diaper topsheets have a basis weight of from about 10 to about 21 gsm, in particular between from about 12 to about 18 gsm but other basis weights are possible.

The backsheet is generally that portion of the diaper positioned adjacent the garment-facing surface of the absorbent core 2 and which prevents the exudates absorbed and contained therein from soiling articles such as bedsheets and undergarments. The backsheet is typically substantially impermeable to liquids (e.g. urine).

The backsheet may for example be or comprise a thin plastic film such as a thermoplastic film having a thickness of about 0.012 mm to about 0.051 mm. Other suitable backsheet materials may include breathable materials which permit vapors to escape from the absorbent article while still preventing exudates from passing through the backsheet. Exemplary breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, Va., and sold under the designation EXAIRE, and monolithic films such as manufactured by Clopay Corporation, Cincinnati, Ohio under the name HYTREL blend P18-3097. Preferable remain film materials as described in relevant embodiments herein.

Preferred backsheets herein are low basis weight backsheets meaning that the basis weight thereof is less than 17gsm, preferably from 10gsm to 16gsm, even more preferably from 13gsm to 15gsm, most preferably about 14gsm. This allows for reduced thickness which enables a lower distance between capacitor plates for optimal operation, yet desirable mechanical attributes may be maintained.

In a preferred embodiment the backsheet is breathable. Breathable backsheet herein typically means that it comprises micro-openings sized to allow at least some water vapour to permeate through the backsheet that typically comprises a film such as a polyethylene (PE) film.

The backsheet may be joined to the topsheet, the absorbent core 2 or any other element of the diaper by any attachment means known in the art. Suitable attachment means are described above with respect to means for joining the topsheet to other elements of the diaper. For example, the attachment means may include a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minn. and marketed as HL-1620 and HL 1358-XZP. Alternatively, the attachment means may comprise heat bonds, pressure bonds, ultrasonic bonds, dynamic mechanical bonds, or any other suitable attachment means or combinations of these attachment means as are known in the art.

The absorbent core 2 of the absorbent article may comprise one or more core layers. As explained, the absorbent article may comprise an acquisition distribution system, which will typically consist of one or more layers. Most typically, the layers are arranged above the core layer. Hence, a number of layers can be arranged between the topsheet and the backsheet. The skilled person will usually have no difficulty in distinguishing between these layers. In case of doubt, a core layer can be identified as being a layer which is generally less permeable than a layer forming part of the acquisition-/distribution-system.

Permeability generally refers to the quality of a porous material that causes it to a lower liquid or gases to pass through it. Hence, the layers of the acquisition distribution system should generally be more permeable than the layers of the core system as these layers are meant to distribute liquid to the absorbent core, where the liquid is ultimately stored.

The absorbent core can comprise absorbent material with a varying amount of superabsorbent polymers (herein abbreviated as "SAP"), often enclosed within a core wrap. The SAP content can represent from 0% to 80% by weight of the absorbent material contained in the core wrap. Often an SAP content of 20% to 80% by weight of the absorbent material contained in the core wrap is useful. The core wrap is not considered as absorbent material for the purpose of assessing the percentage of SAP in the absorbent core.

The SAP content may be higher than 30%, for example at least 40%, at least 50%, at least 80% of the weight of the absorbent material contained within the core wrap. The absorbent material may in particular embodiments comprise from 10 to 70, for example 30 to 60 weight percent of natural or synthetic fibers.

The absorbent core may comprise a generally planar top edge and a generally planar bottom edge. In some embodiments, the absorbent material will be advantageously distributed in higher amount towards the front edge than towards the rear edge as more absorbency is required at the front. In other embodiments, typically embodiments for other uses of an absorbent article, such as care of elderly incontinent people versus care of babies, the absorbent material will be advantageously distributed in higher amount towards the rear edge than towards the front edge as more absorbency is required at the rear area.

The core wrap may be formed by two separate sheets of nonwoven material which may be at least partially sealed along the edges of the absorbent core. The core wrap may be at least partially sealed along its front edge, back edge and two longitudinal edges so that substantially no absorbent material leaks out of the absorbent core wrap.

The absorbent core of the absorbent article may further comprise adhesive for example to help immobilizing the SAP within the core wrap and/or to ensure integrity of the core wrap, in particular when the core wrap is made of two or more substrates. Such an adhesive can be provided in the form of fibrous thermoplastic adhesive material.

Adhesive or mechanical bonding (such as ultrasonic bonding, thermal bonding, pressure bonding etc.) may be used for bonding top and bottom core wrap layers to form channels substantially free of absorbent material as described herein.

The fibrous thermoplastic adhesive material may be at least partially in contact with the superabsorbent material in the land areas and at least partially in contact with the substrate layer in the junction areas. This imparts an essentially three-dimensional structure to the fibrous layer of thermoplastic adhesive material, which in itself is essentially a two-dimensional structure of relatively small thickness, as compared to the dimension in length and width directions. Thereby, the fibrous thermoplastic adhesive material may provide cavities to cover the absorbent material in the land area, and thereby immobilizes this absorbent material.

The thermoplastic adhesive material may comprise, in its entirety, a single thermoplastic polymer or a blend of thermoplastic polymers, having a softening point, as determined by the ASTM Method D-36-95 "Ring and Ball", in the range between 50° C. and 300° C., and/or the thermoplastic adhesive material may be a hotmelt adhesive comprising at least one thermoplastic polymer in combination with other thermoplastic diluents such as tackifying resins, plasticizers and additives such as antioxidants.

Superabsorbent material, herein also referred to as superabsorbent polymer material, superabsorbent polymers or SAP, refers to absorbent materials which are cross-linked polymeric materials that can absorb at least 10 times their weight of an aqueous 0.9% saline solution as measured using the Centrifuge Retention Capacity (CRC) test (EDANA method WSP 241.2-05E). The SAP used may in particular have a CRC value of more than 20 g/g, or more than 24 g/g, or of from 20 to 50 g/g, or from 20 to 40 g/g, or 24 to 30 g/g. The SAP useful in example absorbent articles may include a variety of water-insoluble, but water-swellable polymers capable of absorbing large quantities of fluids.

The superabsorbent polymer can be in particulate form so as to be flowable in the dry state. Typical particulate absorbent polymer materials are made of poly(meth)acrylic acid polymers. However, e.g. starch-based particulate absorbent polymer material may also be used, as well polyacrylamide copolymer, ethylene maleic anhydride copolymer, cross-linked carboxymethylcellulose, polyvinyl alcohol copolymers, cross-linked polyethylene oxide, and starch grafted copolymer of polyacrylonitrile. The superabsorbent polymer may be polyacrylates and polyacrylic acid polymers that are internally and/or surface cross-linked.

The SAP useful for example embodiments of absorbent articles may be of numerous shapes. The term "particles" refers to granules, fibers, flakes, spheres, powders, platelets and other shapes and forms known to persons skilled in the art of superabsorbent polymer particles. In some embodiments, the SAP particles can be in the shape of fibers, i.e. elongated, acicular superabsorbent polymer particles. In those embodiments, the superabsorbent polymer particles fibers have a minor dimension (i.e. diameter of the fiber) of less than about 1 mm, usually less than about 500 μm, and preferably less than 250 μm down to 50 μm. The length of the fibers is preferably about 3 mm to about 100 mm. The fibers can also be in the form of a long filament that can be woven.

Typically, SAP are spherical-like particles. In contrast to fibers, "spherical-like particles" have a longest and a smallest dimension with a particulate ratio of longest to smallest particle dimension in the range of 1-5, where a value of 1 would equate a perfectly spherical particle and 5 would allow for some deviation from such a spherical particle. The superabsorbent polymer particles may have a particle size of less than 850 $\mu$m, or from 50 to 850 $\mu$m, preferably from 100 to 710 $\mu$m, more preferably from 150 to 650 $\mu$m, as measured according to EDANA method WSP 220.2-05. SAP having a relatively low particle size help to increase the surface area of the absorbent material which is in contact with liquid exudates and therefore support fast absorption of liquid exudates.

The SAP may have a particle sizes in the range from 45 $\mu$m to 4000 $\mu$m, more specifically a particle size distribution within the range of from 45 $\mu$m to about 2000 $\mu$m, or from about 100 $\mu$m to about 1000, 850 or 600 $\mu$m. The particle size distribution of a material in particulate form can be determined as it is known in the art, for example by means of dry sieve analysis (EDANA 420.02 "Particle Size distribution).

The total amount of SAP present in the absorbent core may also vary according to expected usage. Diapers for newborns may require less SAP than infant or adult incontinence diapers. The amount of SAP in the core may be for example comprised from about 2 to 60 g, in particular from 5 to 50 g or 10 to 40 g. The average SAP basis weight within the (or "at least one", if several are present) deposition area of the SAP may be for example of at least 50, 100, 200, 300, 400, 500 or more g/m2. The areas of the channels present in the absorbent material deposition area, if any, are deduced from the absorbent material deposition area to calculate this average basis weight.

The optional core wrap may be made of a single substrate folded around the absorbent material, or may advantageously comprise two (or more) substrates which are attached to another. Typical attachments are the so-called C-wrap and/or sandwich wrap. In a C-wrap, the longitudinal and/or transversal edges of one of the substrate are folded over the other substrate to form flaps. These flaps are then bonded to the external surface of the other substrate, typically by gluing.

The absorbent core may comprise one or more channels substantially free of absorbent material and that may be substantially circumscribed by absorbent material such that they do not extend to one or more of the perimeter edges of said core. Top and bottom core wrap layers may be joined together in one or more attachment zones to form said channels. Advantageously such channels aid to improve liquid distribution over the core.

In a preferred embodiment, top and bottom layers of a core wrap are joined together to form a pocket substantially free of absorbent material. Similar to channels described above, the pocket is preferably (but not necessarily) substantially circumscribed by absorbent material such that they do not extend to one or more of the perimeter edges of said core. The pocket may have a geometry with an aspect ratio approaching 1 (such as from 0.5 to less than 2). Advantageously such pocket allows the stool to be collected and with the weight thereof be pushed closer to the backsheet compared to a core in absence of such pocket (generally in view of the reduced thickness in that region by the fact that substantially no absorbent material is present).

In a preferred embodiment, the pocket is positioned such that it substantially superposes the second indicator (4). Advantageously this allows to reduce the power source required to generated the electric field desired for stool detection by e.g. capacitive measurements.

If the core wrap comprises a first substrate and a second substrate these may be made of the same type of material, or may be made of different materials or one of the substrate may be treated differently than the other to provide it with different properties. As the polymers used for nonwoven production are inherently hydrophobic, they are preferably coated with hydrophilic coatings if placed on the fluid receiving side of the absorbent core. It is advantageous that the top side of the core wrap, i.e. the side placed closer to the wearer in the absorbent article, be more hydrophilic than the bottom side of the core wrap. A possible way to produce nonwovens with durably hydrophilic coatings is via applying a hydrophilic monomer and a radical polymerization initiator onto the nonwoven, and conducting a polymerization activated via UV light resulting in monomer chemically bound to the surface of the nonwoven. An alternative possible way to produce nonwovens with durably hydrophilic coatings is to coat the nonwoven with hydrophilic nanoparticles.

The absorbent article may comprise a pair of barrier leg cuffs. The barrier leg cuffs can be formed from a piece of material, typically a nonwoven, which is partially bonded to the rest of the article so that a portion of the material, the barrier leg cuffs, can be partially raised away and stand up from the plane defined by the topsheet when the article is pulled flat as. The barrier leg cuffs can provide improved containment of liquids and other bodily exudates approximately at the junction of the torso and legs of the wearer.

The barrier leg cuffs can be integral with the topsheet or the backsheet, or more typically be formed from a separate material joined to the rest of the article. Typically the material of the barrier leg cuffs may extend through the whole length of the diapers but is "tack bonded" to the topsheet towards the front edge and back edge of the article so that in these sections the barrier leg cuff material remains flush with the topsheet. Each barrier leg cuff may comprise one, two or more elastic close to this free distal edge to provide a better seal.

In addition to the barrier leg cuffs, the article may comprise gasketing cuffs, which are joined to the chassis of the absorbent, article, in particular the topsheet and/or the backsheet and are placed transversely outwardly relative to the barrier leg cuffs. The gasketing cuffs can provide a better seal around the thighs of the wearer. Usually each gasketing leg cuff will comprise one or more elastic string or elastic element comprised in the chassis of the diaper for example between the topsheet and backsheet in the area of the leg openings.

Embodiments of the absorbent articles may comprise an acquisition-distribution layer or system (herein "ADL") and may be a single layer (preferably a spunbond or carded thermobonded layer, or an air-layed nonwoven layer) or may be multilayer and comprising one or more of the following nonwovnen layers: spunbond, carded thermobonded, meltblown, and combinations thereof. The function of the ADL is to quickly acquire the fluid and distribute it to the absorbent core in an efficient manner. The ADL may comprise one, two or more layers, which may form a unitary layer or remain discrete layers which may be attached to each other. In the examples below, the ADL can comprise two layers: a distribution layer and an acquisition layer disposed between the absorbent core and the topsheet, but embodiments of absorbent articles are not restricted to this example and rather multilayer or single-layer ADLs are equally contemplated herein.

Typically, the ADL will not comprise SAP as this may slow the acquisition and distribution of the fluid.

The distribution layer and the acquisition layer may comprise the same or different layers. For example the distribution layer may be a nonwoven selected from a spunbond, meltblown, carded (preferably thermobonded), and combinations therof.

The ADL may comprise an acquisition layer. The acquisition layer may be disposed between the distribution layer and topsheet. The acquisition layer may typically be or comprise a non-woven material, for example a SMS or SMMS material, comprising a spunbonded, a melt-blown and a further spunbonded layer or alternatively a carded chemical-bonded nonwoven.

The absorbent article may include a fastening system. The fastening system can be used to provide lateral tensions about the circumference of the absorbent article to hold the absorbent article on the wearer as is typical for taped diapers. This fastening system is not necessary for training pant article since the waist region of these articles is already bonded. The fastening system usually comprises a fastener such as tape tabs (also referred to as adhesive tabs), hook and loop fastening components, interlocking fasteners such as tabs & slots, buckles, buttons, snaps, and/or hermaphroditic fastening components, although any other known fastening means are generally acceptable. A landing zone is normally provided on the front waist region for the fastener to be releasably attached.

The absorbent article may comprise front ears and back ears as is known in the art. The ears can be integral part of the chassis, for example formed from the topsheet and/or backsheet as side panel. Alternatively, they may be separate elements attached by gluing and/or heat embossing or pressure bonding. The back ears are advantageously stretchable to facilitate the attachment of the adhesive tabs on the landing zone and maintain the taped diapers in place around the wearer's waist. The back ears may also be elastic or extensible to provide a more comfortable and contouring fit by initially conformably fitting the absorbent article to the wearer and sustaining this fit throughout the time of wear well past when absorbent article has been loaded with exudates since the elasticized ears allow the sides of the absorbent article to expand and contract.

The absorbent article may also comprise at least one elastic waist feature that helps to provide improved fit and containment. The elastic waist feature is generally intended to elastically expand and contract to dynamically fit the wearer's waist. The elastic waist feature preferably extends at least longitudinally outwardly from at least one waist edge of the absorbent core 2 and generally forms at least a portion of the end edge of the absorbent article. Disposable diapers can be constructed so as to have two elastic waist features, one positioned in the front waist region and one positioned in the back waist region. The elastic waist feature may be constructed in a number of different configurations including those described in U.S. Pat. Nos. 4,515,595, 4,710,189, 5,151,092 and 5,221,274.

Typically, adjacent layers and components will be joined together using conventional bonding method such as adhesive coating via slot coating or spraying on the whole or part of the surface of the layer, or thermo-bonding, or pressure bonding or combinations thereof.

## KIT OF PARTS

In a further aspect the disclosure relates to a kit comprising a plurality of absorbent articles herein described and a detection device comprising the second indicator and operable with any one of said absorbent articles. Preferably wherein the plurality of absorbent articles comprises absorbent articles having at least two, preferably at least three, different absorbancy levels and/or sizes. Advantageously this allows for ease of use and replenishment for stock management purposes and the use of a same detection device on multiple absorbent articles that may be assigned to a patient and/or user.

## Claims

1. An absorbent article (1) for personal hygiene comprising:

   a topsheet (TS);
   a backsheet (BS), wherein the topsheet is liquid permeable and the backsheet is substantially liquid impermeable;
   an absorbent core (2) positioned between said topsheet and backsheet,

a first indicator (3) for indicating the presence of exudates and being positioned on a garment-facing side of said backsheet such that said first indicator is sandwiched between said backsheet (BS) and an outer cover (OC) of said article (1); or wherein said first indicator (3) is positioned on a body-facing side of said backsheet such that said backsheet and an outer cover (OC) are able to come into contact with one another;

and wherein said first indicator (3) comprises an electrically conductive material, wherein the first indicator (3) comprises one or more first input capacitors (3i), one or more first output capacitors (3ii) and a plurality of conductive tracks (3iii) forming an electrical connection between the input and output capacitors,

**characterized in that** a reusable or single-use second indicator (4) is arranged to be capacitively coupled with at least the first input and output capacitors such that upon a voiding event an amount of exudate may be automatically determined by measuring a dissipation factor or attenuation of an input signal, said second indicator (4) comprising one or more second input capacitors (4i), one or more second output capacitors (4ii) and a sensing circuitry (SC), and **in that** the backsheet (BS) is breathable and has a water vapour transmission rate (WVTR) of at least 3000g/(m$^2$.24h), according to the method described herein; and/or wherein the outer cover (OC) is a nonwoven comprising synthetic fibers.

2. An absorbent article (1) according to Claim 1 wherein the water vapour transmission rate (WVTR) is at least 3500 g/(m$^2$.24h), preferably at least 4000 g/(m$^2$.24h), preferably grater than 4000 g/(m$^2$.24h), preferably from 4500 g/(m$^2$.24h) to 10000 g/(m$^2$.24h), preferably from 5000 g/(m$^2$.24h) to 9000 g/(m$^2$.24h), even more preferably from 5500 g/(m$^2$.24h) to 8500 g/(m$^2$.24h), even more preferably from 6000 g/(m$^2$.24h) to 8200 g/(m$^2$.24h), according to the method herein.

3. An absorbent article (1) according to any of the preceding Claims wherein the second indicator (4) is in the form of a re-usable sensor (S) adapted to be joined to the outer cover (OC) of the absorbent article by means of a releasable coupling.

4. An absorbent article according to any of the preceding Claims, wherein the second indicator (4) is in electrical communication with a power source providing an alternating current and at least in data or signal communication with a processing unit.

5. An absorbent article according to any of the preceding Claims wherein the conductive tracks undergo a change in one or more electric properties in response to a voiding event such as direct or indirect contact with exudates and/or moisture caused therefrom.

6. An absorbent article according to any of the preceding Claims wherein the outer cover (OC) is a non-woven comprising, preferably consisting of, synthetic fibers and wherein said fibers comprise, preferably consist of, polyprolylene; and/or wherein the outer cover (OC) is a nonwoven having a basis weight of from 5gsm to 25gsm, preferably from 6gsm to 20gsm, even more preferably from 7gsm to 18gsm.

7. An absorbent article according to Claim 6 wherein the outer cover (OC) is a spunbond nonwoven or a laminate comprising one or more spunbond nonwoven layers.

8. An absorbent article according to any of the preceding Claims wherein the first indicator (3) is printed with a conductive ink, preferably wherein the conductive ink is a carbon based ink and/or a conductive polymer based ink.

9. An absorbent article according to any of the preceding Claims wherein a total surface area of the first indicator (3) is greater than a total surface area of the second indicator, wherein said surface area is taken about a plane corresponding to the backsheet (BS), preferably wherein the total surface area of the first indicator (3) is more than 2 times, preferably more than 3 times, even more preferably from 4 to 10 times, the total surface area of the second indicator (4).

10. An absorbent article according to any of the preceding Claims wherein the first and second indicators (3, 4) have different shapes, preferably wherein the first indicator comprises a first shape and the second indicator comprises a second shape and wherein the second shape overlaps a portion of the first shape with portions of the first shape extending inboard and/or outboard of the second shape.

11. An absorbent article according to any of the preceding Claims wherein the second indicator is coated and/or contained within a dielectric sleeve (6), preferably said coating and/or sleeve comprises silicone.

12. A system for sensing exudates comprising:

- an absorbent article according to any of the preceding Claims; and
- an external sensing unit, wherein said external sensing unit comprises the second indicator (4) according to any of the preceding Claims;

wherein said external sensing unit is releasably coupled with the absorbent article.

13. A system according to Claim 12 adapted to measure a dissipation factor or a signal attenuation between input and output signals corresponding to a power loss and/or voltage-drop caused by a change of resistance along one or more of the conductive tracks of the first indicator (3).

14. A system according to any of Claims 12 to 13 wherein the second indicator (4) of the external sensing unit is positioned at a distance in a thickness direction from the first indicator (3) and wherein said distance is less than 5mm, preferably from 0.5mm to 3mm, even more preferably from 0.8mm to 2.5mm.

15. A system according to any of the preceding Claims comprising a plurality of detection zones wherein each detection zone corresponds to a pair of consecutive tracks connecting respective first input and output capacitors and wherein at least one of the first input capacitors and at least one of the first output capacitors serve as a common electrode junction.

**Patentansprüche**

1. Absorbierender Artikel (1) für Körperhygiene, umfassend:

eine obere Lage (TS);
eine untere Lage (BS), wobei die obere Lage flüssigkeitsdurchlässig und die untere Lage im Wesentlichen flüssigkeitsundurchlässig ist;
einen absorbierenden Kern (2), der zwischen der oberen Lage und der unteren Lage positioniert ist,
einen ersten Indikator (3) zum Indizieren des Vorhandenseins von Exsudaten und der auf einer der Kleidung zugewandten Seite der unteren Lage derart positioniert ist, dass der erste Indikator zwischen der unteren Lage (BS) und einer äußeren Abdeckung (OC) des Artikels (1) eingebettet ist; oder wobei der erste Indikator (3) auf einer dem Körper zugewandten Seite der unteren Lage derart positioniert ist, dass die untere Lage und eine äußere Abdeckung (OC) miteinander in Berührung kommen können;
und wobei der erste Indikator (3) ein elektrisch leitfähiges Material umfasst, wobei der erste Indikator (3) einen oder mehrere erste Eingangskondensatoren (3i), einen oder mehrere erste Ausgangskondensatoren (3ii) und eine Vielzahl von leitfähigen Bahnen (3iii) umfasst, die eine elektrische Verbindung zwischen den Eingangs- und Ausgangskondensatoren ausbilden,
**dadurch gekennzeichnet, dass** ein wiederverwendbarer oder zum Einmalgebrauch vorgesehener zweiter Indikator (4) angeordnet ist, um mit mindestens dem ersten Eingangs- und Ausgangskondensator derart kapazitiv gekoppelt zu sein, dass bei einem Entleerungsereignis eine Exsudatmenge durch Messen eines Verlustfaktors oder einer Dämpfung eines Eingangssignals automatisch bestimmt werden kann, der zweite Indikator (4) umfassend einen oder mehrere zweite Eingangskondensatoren (4i), einen oder mehrere zweite Ausgangskondensatoren (4ii) und eine Abtastschaltung (SC), und **dass** die untere Lage (BS) atmungsaktiv ist und eine Wasserdampfdurchlässigkeit (WVTR) von mindestens 3000 g/(m$^2$.24h) nach dem hierin beschriebenen Verfahren aufweist; und/oder wobei die äußere Abdeckung (OC) ein Vlies ist, umfassend synthetische Fasern.

2. Absorbierender Artikel (1) nach Anspruch 1, wobei die Wasserdampfdurchlässigkeitsrate (WVTR) mindestens 3500 g/(m$^2$.24h), vorzugsweise mindestens 4000 g/(m$^2$.24h), vorzugsweise größer als 4000 g/(m$^2$.24h), vorzugsweise von 4500 g/(m$^2$.24h) bis 10000 g/(m$^2$.24h), vorzugsweise von 5000 g/(m$^2$.24h) bis 9000 g/(m$^2$.24h), noch mehr bevorzugt von 5500 g/(m$^2$.24h) bis 8500 g/(m$^2$.24h), noch mehr bevorzugt von 6000 g/(m$^2$.24h) bis 8200 g/(m$^2$.24h), nach dem hierin beschriebenen Verfahren.

3. Absorbierender Artikel (1) nach einem der vorstehenden Ansprüche, wobei der zweite Indikator (4) in der Form eines wiederverwendbaren Sensors (S) vorliegt, der geeignet ist, um mittels einer lösbaren Kupplung mit der äußeren Abdeckung (OC) des absorbierenden Artikels zusammengefügt zu werden.

4. Absorbierender Artikel nach einem der vorstehenden Ansprüche, wobei der zweite Indikator (4) in elektrischer Kommunikation mit einer Stromquelle steht, die einen Wechselstrom bereitstellt, und mindestens in Daten- oder Signalkommunikation mit einer Verarbeitungseinheit steht.

5. Absorbierender Artikel nach einem der vorstehenden Ansprüche, wobei die leitfähigen Bahnen als Reaktion auf ein Entleerungsereignis, wie direkte oder indirekte Berührung mit Exsudaten und/oder daraus verursachter Feuchtigkeit, eine Änderung einer oder mehrerer elektrischer Eigenschaften erfahren.

6. Absorbierender Artikel nach einem der vorstehenden Ansprüche, wobei die äußere Abdeckung (OC) ein Vlies ist, umfassend, vorzugsweise bestehend aus synthetische(n) Fasern, und wobei die Fasern Polypropylen umfassen, vorzugsweise daraus bestehen; und/oder wobei die äußere Abdeckung (OC) ein Vlies ist, das ein Basisgewicht von 5 g/m² bis 25 g/m², vorzugsweise von 6 g/m² bis 20 g/m², noch mehr bevorzugt von 7 g/m² bis 18 g/m² aufweist.

7. Absorbierender Artikel nach Anspruch 6, wobei die äußere Abdeckung (OC) ein Spinnvlies oder ein Laminat ist, umfassend eine oder mehrere Spinnvliesschichten.

8. Absorbierender Artikel nach einem der vorstehenden Ansprüche, wobei der erste Indikator (3) mit einer leitfähigen Tinte bedruckt ist, vorzugsweise wobei die leitfähige Tinte eine Tinte auf Kohlenstoffbasis und/oder eine leitfähige Tinte auf Polymerbasis ist.

9. Absorbierender Artikel nach einem der vorstehenden Ansprüche, wobei ein Gesamtoberflächenbereich des ersten Indikators (3) größer als eine Gesamtoberfläche des zweiten Indikators ist, wobei der Oberflächenbereich um eine Ebene herum gemessen wird, die der unteren Lage (BS) entspricht, vorzugsweise wobei der Gesamtoberflächenbereich des ersten Indikators (3) mehr als das Zweifache, vorzugsweise mehr als das Dreifache, noch mehr bevorzugt das Vier- bis Zehnfache des Gesamtoberflächenbereichs des zweiten Indikators (4) beträgt.

10. Absorbierender Artikel nach einem der vorstehenden Ansprüche, wobei der erste und der zweite Indikator (3, 4) unterschiedliche Formen aufweisen, vorzugsweise wobei der erste Indikator eine erste Form umfasst und der zweite Indikator eine zweite Form umfasst, und wobei die zweite Form einen Abschnitt der ersten Form überlappt, wobei Abschnitte der ersten Form sich nach innen und/oder außen von der zweiten Form erstrecken.

11. Absorbierender Artikel nach einem der vorstehenden Ansprüche, wobei der zweite Indikator beschichtet und/oder innerhalb einer dielektrischen Hülle (6) enthalten ist, vorzugsweise wobei die Beschichtung und/oder Hülle Silikon umfasst.

12. System zum Abtasten von Exsudaten, umfassend:

- einen absorbierenden Artikel nach einem der vorstehenden Ansprüche; und
- eine externe Abtasteinheit, wobei die externe Abtasteinheit den zweiten Indikator (4) gemäß einem der vorstehenden Ansprüche umfasst;

wobei die externe Abtasteinheit mit dem absorbierenden Artikel lösbar gekoppelt ist.

13. System nach Anspruch 12, das geeignet ist, um einen Verlustfaktor oder eine Signaldämpfung zwischen Eingangs- und Ausgangssignalen zu messen, die einem Leistungsverlust und/oder einem Spannungsabfall entspricht, der durch eine Widerstandsänderung entlang einer oder mehrerer leitfähiger Bahnen des ersten Indikators (3) verursacht wird.

14. System nach einem der Ansprüche 12 bis 13, wobei der zweite Indikator (4) der externen Abtasteinheit in einer Dickenrichtung in einem Abstand von dem ersten Indikator (3) positioniert ist und wobei der Abstand weniger als 5 mm, vorzugsweise von 0,5 mm bis 3 mm, noch mehr bevorzugt von 0,8 mm bis 2,5 mm beträgt.

15. System nach einem der vorstehenden Ansprüche, umfassend eine Vielzahl von Erfassungszonen, wobei jede Erfassungszone einem Paar aufeinanderfolgender Bahnen entspricht, die jeweilige erste Eingangs- und Ausgangskondensatoren verbinden, und wobei mindestens einer der ersten Eingangskondensatoren und mindestens einer der ersten Ausgangskondensatoren als eine Verbindungsstelle gemeinsamer Elektroden dienen.

## Revendications

1. Article absorbant (1) destiné à l'hygiène personnelle comprenant :

une feuille de couverture (TS) ;
une feuille arrière (BS), dans lequel la feuille de couverture est perméable aux liquides et la feuille arrière est pratiquement imperméable aux liquides ;
une âme absorbante (2) placée entre ladite feuille de couverture et ladite feuille arrière,
un premier indicateur (3) destiné à indiquer la présence d'exsudats et placé sur une face de ladite feuille arrière orientée vers le vêtement, de telle sorte que ledit premier indicateur est pris en sandwich entre ladite feuille arrière (BS) et une couverture extérieure (OC) dudit article (1) ;
ou dans lequel ledit premier indicateur (3) est placé sur une face de ladite feuille arrière orientée vers le corps, de telle sorte que ladite feuille arrière et une couverture extérieure (OC) peuvent entrer en contact l'une avec l'autre ;
et dans lequel ledit premier indicateur (3) comprend un matériau électriquement conducteur, dans lequel le premier indicateur (3) comprend un ou plusieurs premiers condensateurs d'entrée (3i), un ou plusieurs premiers condensateurs de sortie (3ii) et une pluralité de pistes conductrices (3iii) formant une connexion électrique entre les condensateurs d'entrée et de sortie,

**caractérisé en ce qu'**un second indicateur (4) réutilisable ou à usage unique est agencé pour être couplé de manière capacitive avec au moins les premiers condensateurs d'entrée et de sortie de telle sorte que, lors d'un événement d'évacuation, une quantité d'exsudat peut être automatiquement déterminée en mesurant un facteur de dissipation ou d'atténuation d'un signal d'entrée, ledit second indicateur (4) comprenant un ou plusieurs seconds condensateurs d'entrée (4i), un ou plusieurs seconds condensateurs de sortie (4ii) et une circuiterie de détection (SC), et **en ce que** la feuille arrière (BS) est respirante et a un taux de transmission de vapeur d'eau (WVTR) d'au moins 3 000 g/(m$^2$.24h), selon le procédé décrit ici ; et/ou dans lequel la couverture extérieure (OC) est un non-tissé comprenant des fibres synthétiques.

2. Article absorbant (1) selon la revendication 1, dans lequel le taux de transmission de vapeur d'eau (WVTR) est d'au moins 3 500 g/(m$^2$.24h), de préférence d'au moins 4 000 g/(m$^2$.24h), de préférence supérieur à 4 000 g/(m$^2$.24h), de préférence de 4 500 g/(m$^2$.24h) à 10 000 g/(m$^2$.24h), de préférence de 5 000 g/(m$^2$.24h) à 9 000 g/(m$^2$.24h), encore plus préférablement de 5 500 g/(m$^2$.24h) à 8 500 g/(m$^2$.24h), encore plus préférablement de 6 000 g/(m$^2$.24h) à 8 200 g/(m$^2$.24h), selon le présent procédé.

3. Article absorbant (1) selon l'une quelconque des revendications précédentes, dans lequel le second indicateur (4) se présente sous la forme d'un capteur réutilisable (S) adapté pour être relié à l'enveloppe extérieure (OC) de l'article absorbant au moyen d'un raccord amovible.

4. Article absorbant selon l'une quelconque des revendications précédentes, dans lequel le second indicateur (4) est en communication électrique avec une source de puissance fournissant un courant alternatif et au moins en communication de données ou par signal avec une unité de traitement.

5. Article absorbant selon l'une quelconque des revendications précédentes, dans lequel les pistes conductrices subissent une modification dans une ou plusieurs propriétés électriques en réponse à un événement d'évacuation tel qu'un contact direct ou indirect avec des exsudats et/ou l'humidité qui en résulte.

6. Article absorbant selon l'une quelconque des revendications précédentes, dans lequel la couverture extérieure (OC) est un non-tissé comprenant, de préférence étant constitué, des fibres synthétiques et dans lequel lesdites fibres comprennent, de préférence étant constituées, du polyprolylène ; et/ou dans lequel la couverture extérieure (OC) est un non-tissé ayant un poids de base de 5 g/m$^2$ à 25 g/m$^2$, de préférence de 6 g/m$^2$ à 20 g/m$^2$, encore plus préférablement de 7 g/m$^2$ à 18 g/m$^2$.

7. Article absorbant selon la revendication 6, dans lequel la couverture extérieure (OC) est un non-tissé filé-lié ou un laminé comprenant une ou plusieurs couches de non-tissé filé-lié.

8. Article absorbant selon l'une quelconque des revendications précédentes, dans lequel le premier indicateur (3) est imprimé avec une encre conductrice, de préférence dans lequel l'encre conductrice est une encre à base de carbone et/ou une encre conductrice à base de polymère.

9. Article absorbant selon l'une quelconque des revendications précédentes, dans lequel une surface totale du premier indicateur (3) est supérieure à une surface totale du second indicateur, dans lequel ladite surface est prise autour d'un plan correspondant à la feuille arrière (BS), de préférence dans lequel la surface totale du premier indicateur (3) est plus de 2 fois, de préférence plus de 3 fois, encore plus préférablement de 4 à 10 fois, la surface totale du second indicateur (4).

10. Article absorbant selon l'une quelconque des revendications précédentes, dans lequel les premier et second indicateurs (3, 4) ont des formes différentes, de préférence dans lequel le premier indicateur comprend une première forme et le second indicateur comprend une seconde forme et dans lequel la seconde forme chevauche une partie de la première forme avec des parties de la première forme s'étendant vers l'intérieur et/ou vers l'extérieur de la seconde forme.

11. Article absorbant selon l'une quelconque des revendications précédentes, dans lequel le second indicateur est revêtu et/ou contenu dans un manchon diélectrique (6), de préférence ledit revêtement et/ou manchon comprend du silicone.

12. Système de détection d'exsudats comprenant :

    - un article absorbant selon l'une quelconque des revendications précédentes ; et
    - une unité de détection externe, dans lequel ladite unité de détection externe comprend le second indicateur (4) selon l'une quelconque des revendications précédentes ;

dans lequel ladite unité de détection externe est couplée de manière amovible à l'article absorbant.

13. Système selon la revendication 12, adapté pour mesurer un facteur de dissipation ou une atténuation de signal entre des signaux d'entrée et de sortie correspondant à une perte de puissance et/ou une chute de tension causée par un changement de résistance le long d'une ou plusieurs pistes conductrices du premier indicateur (3).

14. Système selon l'une quelconque des revendications 12 à 13, dans lequel le second indicateur (4) de l'unité de détection externe est positionné à une certaine distance du premier indicateur (3) dans une direction d'épaisseur, et dans lequel ladite distance est inférieure à 5 mm, de préférence de 0,5 mm à 3 mm, encore plus préférablement de 0,8 mm à 2,5 mm.

15. Système selon l'une quelconque des revendications précédentes comprenant une pluralité de zones de détection, dans lequel chaque zone de détection correspond à une paire de pistes consécutives connectant des premiers condensateurs d'entrée et de sortie respectifs et dans lequel au moins l'un parmi les premiers condensateurs d'entrée et au moins l'un parmi les premiers condensateurs de sortie servent de jonction d'électrode commune.

FIG. 1

FIG. 2

FIG. 3

FIG. 4

**FIG. 5**

FIG. 6

FIG. 7

**FIG. 8**

FIG. 9

FIG. 10

EP 4 548 887 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5151092 A, Buell
- WO 2008155699 A
- WO 2012052172 A
- EP 2496197 B1
- EP 2739254 B1
- EP 2582341 B1
- EP 3415130 A
- WO 2018228822 A
- WO 2018229017 A
- EP 3461257 A
- EP 3451988 A
- WO 2022136295 A1
- EP 3906907 A1
- US 6632504 B
- WO 2011163582 A
- US 4515595 A
- US 4710189 A
- US 5221274 A